# EUROPEAN PATENT SPECIFICATION

(11) **EP 2 084 137 B1**
(45) Date of publication and mention of the grant of the patent: **24.04.2013**
(21) Application number: 07822223.9
(22) Date of filing: 05.11.2007
(51) Int. Cl.: C07D 231/44, A01N 43/56, A01N 43/00, A61K 31/415, A61P 33/00

(54) **PROCESS FOR THE SULFINYLATION OF A PYRAZOLE DERIVATIVE**
VERFAHREN ZUR SULFINYLIERUNG EINES PYRAZOLDERIVATS
PROCÉDÉ DE SULFINYLATION D'UN DÉRIVÉ PYRAZOLE

(30) Priority: 10.11.2006 US 865178 P; 24.04.2007 US 913646 P
(43) Date of publication of application: 05.08.2009
(73) Proprietor: BASF Agro B.V., Arnhem (NL), Zürich Branch, 8055 Zürich (CH)
(72) Inventor: SUKOPP, Martin, 68165 Mannheim (DE); KUHN, Oliver, 06581 Rosport (LU); GRÖNING, Carsten, 68259 Mannheim (DE); KEIL, Michael, 67251 Freinsheim (DE); LONGLET, Jon J., Nederland, TX 77627 (US)
(74) Representative: Niedenbrück, Matthias
(86) International application number: PCT/EP2007/061894
(87) International publication number: WO 2008/055880

(56) References cited:
- EP-A- 0 295 117
- CN-A- 1 374 298
- US-A- 5 618 945

## Description

The present invention relates to a novel process for the sulfinylation of a pyrazole derivative, characterized in that 5-amino-1-[2,6-dichloro-4-(trifluoromethyl)phenyl]-1H-pyrazole-3-carbonitrile (II) is reacted with a sulfinylating agent S in the presence of at least one amine acid complex wherein the amine(s) are selected from secondary and/or tertiary amines and the acid(s) are selected from hydrofluoric, hydrochloric, hydrobromic and hydroiodic acid and sulfonic acid derivatives, and with the addition of a halogenating agent, wherein
S is [CF₃S(O)]₂O; or
CF₃S(O)X wherein
X means fluoro, chloro, bromo, iodo, a hydroxy group, or an alkaline or alkaline earth metal salt of the hydroxy group; or
mixtures thereof,
wherein the temperature of the reaction mixture at no time exceeds 39°C.

The sulfinylation of a pyrazole-type compound refers to the substitution of a hydrogen atom on a pyrazole heterocycle carbon atom by an RS(=O)- group.

The direct sulfinylation of various organic molecules (not including pyrazole derivatives) employing a mixture of P(O)Cl₃ and CF₃S(O)ONa has been described in T. Billard, A. Greiner, B. R. Langlois, Tetrahedron 55 (1999), p. 7243 - 7250. Likewise, C. Wakselman, M. Tordeux, C. Freslon, L. Saint-Jalmes, Synlett 2001, p. 550-552 teaches that direct sulfinylation of aromatic compounds takes place by CF₃S(O)ONa or CF₃S(O)OK in the presence of triflic acid (CF₃S(O)₂OH).

Processes for the direct sulfinylation of 5-amino-1-[2,6-dichloro-4-(trifluoromethyl)phenyl]-1H-pyrazole-3-carbonitrile (II) have been described in EP-A 668 269, EP-A 1 331 222, CN-A 1374298, and in Y.Huilong, M. Zengeng, W. Shujuan, J. Hebei University of Science of Technology, Vol. 25(2), Sum 69 (2004), Serial no. 1008-1542 (2004) 02-0018-03.

In EP 668 269, the sulfinylation of 5-amino-1-[2,6-dichloro-4-(trifluoromethyl)phenyl]-1H-pyrazole-3-carbonitrile (II) with trifluoromethylsulfinic acid CF₃S(O)OH and its derivatives CF₃S(O)Cl, CF₃S(O)ONa, CF₃S(O)N(CH₃)₂, or CF₃S(O)N(CH₂CH₃)₂ has been described. As chlorinating agent, phosgene, chloroformates, PCl₅ and SOCl₂ are mentioned. It is described that a reagent ("compound C") chosen from the group consisting of the tosylates, hydrochlorides and mesylates of a primary, secondary, or tertiary amine, preferably of dimethylamine, of pyridine, of trimethylamine, of diethylamine or of isopropylamine or gaseous hydrogen chloride, optionally in the presence of an equimolar amount of para-toluenesulfonic acid, may be added to complete the reaction. The reaction can be conducted at temperatures of 30 to 55°C. Examples are given for the following combinations of reactants and temperatures:
CF₃S(O)Cl, dimethylamine p-tosylate, 50°C;
CF₃S(O)Cl, pyridine hydrochloride salt, 50°C;
CF₃S(O)N(CH₃)₂, p-toluolsulfonic acid, hydrochloric acid, 50°C;
CF₃S(O)Cl, dimethylamine p-tosylate, hydrochloric acid, 50°C; and
CF₃S(O)ONa, dimethylamine p-tosylate, SOCl₂; according to this particular example, SOCl₂ is first added at 5°C, then the reaction is kept at room temperature for several hours, after which the temperature is raised to 50°C. The reactions carried out with CF₃S(O)Cl as the sulfinylating agent give the highest yield of the final product.

The process described in CN-A 1374298 has been developed to overcome certain shortcomings of the process described in EP 668 269. CN-A 1374298 cites that CF₃S(O)Cl is extremely unstable, CF₃S(O)N(CH₃)₂ and CF₃SOOH are relatively difficult to prepare, that the reactivity CF₃S(O)ONa is not high, and that the yield in the sulfinylation reaction is correspondingly relatively low. CN-A 1374298 describes the sulfinylation of 5-amino-1-[2,6-dichloro-4-(trifluoromethyl)phenyl]-1H-pyrazole-3-carbonitrile (II) with the potassium salt of trifluoromethylsulfinic acid, CF₃S(O)OK, or mixtures of the potassium and the sodium salt of trifluoromethylsulfinic acid, CF₃S(O)OK with CF₃S(O)ONa, wherein the sulfinylating agent is combined with POCl₃, PCl₃, SOCl₂, COCl₂, or trichloromethylchloromethanoate. Optionally, the amine acid complex dimethylamine p-tosylate can be added to complete the reaction.
Examples are given for the following combinations of reactants:
CF₃S(O)OK; dimethylamine p-tosylate; POCl₃; and
CF₃S(O)OK/Na; dimethylamine p-tosylate; SOCl₂.
Temperatures of the example reaction mixtures are given to have been between 40°C and 60°C.

Huilong et al. describes the reaction of 5-amino-1-[2,6-dichloro-4-(trifluoromethyl)phenyl]-1H-pyrazole-3-carbonitrile (II) with the sodium salt of trifluoromethylsulfinic acid (CF₃S(O)ONa), dimethylamine p-tosylate, and SOCl₂, with addition of catalytic amounts of DMF (dimethylformamide). The temperature was kept at 3°C for 10 minutes, after which it was raised to room temperature (1 hour) and then to 50°C (10 hours).

As described in EP-A 1 331 222, 5-amino-1-[2,6-dichloro-4-(trifluoromethyl)phenyl]-1H-pyrazole-3-carbonitrile (II) is sulfinylated using N-trifluoromethylsulfinylsuccinimide as the sulfinylating agent in the presence of triethylamine and without the addition of a chlorinating agent, with the temperature being raised from 10°C to room temperature. The intermediate N-trifluoromethylsulfinylamino-pyrazole is isolated and under the conditions of a Thia-Fries rearrangement under tempertures of 35°C to 55°C transformed into the final product 5-amino-1-[2,6-dichloro-4-(trifluoromethyl)phenyl]-4-(trifluoromethylsulfinyl)-pyrazole-3-carbonitrile.

Thus, the sulfinylation of 5-amino-1-[2,6-dichloro-4-(trifluoromethyl)phenyl]-1H-pyrazole-3-carbonitrile (II) to the final product 5-amino-1-[2,6-dichloro-4-(trifluoromethyl)phenyl]-4-(trifluoromethylsulfinyl)-pyrazole-3-carbonitrile (common name: fipronil) has received considerable attention in the literature, with the focus being on the optimization of the sulfinylating agent. The improvements of the process described in the literature have employed dimethylamine p-tosylate and temperatures around 50°C as standard reaction conditions.

However, and also as cited in the recent review article "research progress on synthesis of fipronil and its main intermediates", Chinese Journal of Pesticides, 2004, Vol. 43, no.12, 529-531, the sulfinylation of the pyrazole intermediate was still found not to be generally suitable for a large scale industrial production.

The reaction product of the present sulfinylation is fipronil, which is a market insecticide of considerable importance. Generally, technical manufacturing processes of pesticides have to fulfill high requirements with regard to yield and purity of the product, for reasons of profitability, but also, most important, in order to avoid the presence of potentially noxious side products. This is of special relevance for fipronil as it is also used in animal health products and therefore also comes into contact with companion animals.

Furthermore, during the scale up of a process from the laboratory scale to a technical scale, problems may arise that were not as such or to the respective extend forseeable in the laboratory.
- For example, the filling and/or dissolution of voluminous starting materials may take much longer on a big scale than in a small vessel, with the effect that the kinetic of the reaction is significantly changed, and thereby the conversion and the product spectrum.
- Another example that can be mentioned is the appearance of side products that are, due to solubility or texture, difficult to separate from the desired main product on a large scale. Problems with extraction, filtration and clogging of filters may occurr. Insoluble starting materials or reaction (side) products may also challenge the agitation, heat dissipation or pumping thus leading to inhomogenous reaction mixtures.
- Yet another challenge is the control of the course of the reaction temperature in large-scale processes. The temperature rates generally are lower which may have an influence on the side product spectrum. As high reaction temperatures and/or aggressive reaction media may cause corrosion, and also because of economic reasons, moderate reaction conditions (low temperatures as according to the present invention) are preferred.
- Hygroscopic properties of solids can complicate reactions that favorably are conducted under essentially water free conditions. For example, when the process as defined above is conducted using an amine acid complex wherein the acid is H₂SO₄ - and not an acid as defined for the inventive process - the yield of the reaction is extremely poor.
- Nonreactive catalysts - such as tertiary amines, e.g. triethylamine acids- are preferably used in the inventive process in order to avoid side reactions. Specific secondary or primary amines can react with the sulfinylating agent.
- With a view to facilitating the work-up, reagents are preferably used that can be removed by destillation. Solids are removed by washing with acidic or alkaline solvents. It is not advantageous to employ reagents that have phase transfer catalytic properties which may hinder a phase separation during work-up.

Against this background and facing the fact that one of the essential starting materials for the current industrial production of fipronil is CF₃Br (see e.g. WO 01/30760) which exhibits high environmental toxicity and is scheduled for production phase out by the Montreal Protocol on Substances that Deplete the Ozone Layer (it may then be used only as a feedstock material), it was an object of the present invention to develop a new, large scale industrial process for the manufacturing of fipronil which gives fipronil in high purity and yield while avoiding the use of dangerous reagents and avoiding problems with the technical reaction control.

Accordingly, the process defined at the outset was found. The obtained product fipronil is suitable for use as a pesticide for agricultural uses as well as non-crop uses for combating pests. Also, the obtained fipronil is suitable for use in the animal health field for combating animal health pests and parasites, especially for the long lasting protection against fleas and ticks on mammals.

Thus, the current invention also pertains to pesticidal or parasiticidal composition containing 5-amino-1-[2,6-dichiloro-4-(trifluoromethyl)phenyl]-4-(trifluoromethylsulfinyl)-pyrazole-3-carbonitrile as prepared by the inventive process.

Likewise, the present invention relates to a method for the control of insects, acarids or nematodes by contacting the insect, acarid or nematode or their food supply, habitat, breeding ground or their locus with a pesticidally effective amount of 5-amino-1-[2,6-dichloro-4-(trifluoromethyl)phenyl]-4-(trifluoromethylsulfinyl)-pyrazole-3-carbonitrile prepared by the inventive process as well as to a method of protecting growing plants from attack or infestation by insects, acarids or nematodes by applying to the foliage or the seeds of the plants, or to the soil or water in which they are growing, a pesticidally effective amount of 5-amino-1-[2,6-dichloro-4-(trifluoromethyl)phenyl}-4-(trifluoromethylsulfinyl)-pyrazole-3-carbonitrile prepared by the inventive process. According to these methods, 5-amino-1-[2,6-dichloro-4-(trifluoromethyl)phenyl]-4-(trifluoromethylsulfinyl)-pyrazole-3-carbonitrile is usually applied in an amount of from 5 g/ha to 2000 g/ha.

Also, the present invention relates to a process for the preparation of a composition for treating, controlling, preventing or protecting animals against infestation or infection by parasites which comprises admixing 5-amino-1-[2,6-dichloro-4-(trifluoromethyl)phenyl]-4-(trifluoromethylsulfinyl)-pyrazole-3-carbonitrile prepared by the inventive process or its enantiomers or veterinarily acceptable salts with a veterinarily acceptable carrier. The composition may either be a concentrate or contains 5-amino-1-[2,6-dichloro-4-(trifluoromethyl)phenyl]-4-(trifluoromethylsulfinyl)-pyrazole-3-carbonitrile in a parasiticidally effective amount.

While there are examples given for certain amine acid complexes added at the beginning or during the course of the sulfinylation reaction, there is no teaching as to the crucial importance of the reaction temperature with regard to reaction control or to the yield and / or especially of the purity of the final product fipronil.

In EP-A1 668 269, a general recommendation is given to conduct the reaction between 0°C and 100°C, preferably between 3°C and 60°C, and most preferably between 30°C and 55°C. According to the example protocols, the reaction temperature in each case reaches 50°C and is held at this point for several hours.

Similarly, in both CN-A 1374298 and Huilong et al., examples are given for a temperature range of 40°C to 60°C (CN-A 1374298) and of 50°C (Huilong et al.).

None of the prior art documents mentions or suggests to conduct the reaction at temperatures below 39°C. In fact, the prior art documents do not even mention the problem of optimizing the control of the reaction in order to obtain high purities of the end product fipronil. Given the fact that temperatures were not given particular attention in the prior art - while e.g. the choice of the sulfinylating agent has been discussed intensively - , it has been particularly surprising to find the strong influence of the reaction temperature on the side product spectrum.

The novel subject of the present invention thus is a novel process for the sulfinylation of a pyrazole derivative, characterized in that 5-amino-1-[2,6-dichloro-4-(trifluoromethyl)phenyl]-1H-pyrazole-3-carbonitrile (II) is reacted with a sulfinylating agent S in the presence of at least one amine acid complex wherein the amine(s) are selected from secondary and/or tertiary amines and the acid(s) are selected from hydrofluoric, hydrochloric, hydrobromic and hydroiodic acid and sulfonic acid derivatives, and with the addition of a halogenating agent, wherein S is as defined at the outset, wherein the temperature of the reaction mixture at no time exceeds 39°C.

A reaction scheme may be depicted as follows:

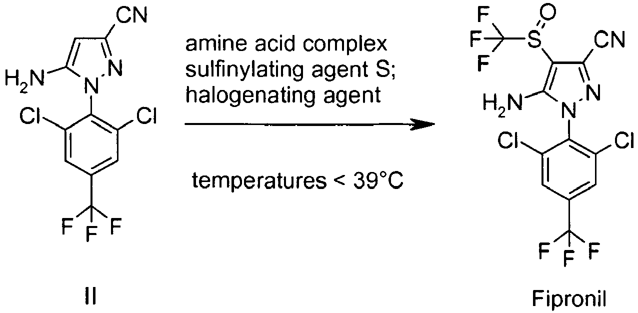

The reaction temperature preferably does not exceed 36°C. The highest temperature preferably is between 25°C and 39°C, even more preferably between 31°C and 39°C, preferably between 32 and 36°C.

The sulfinylating agent is preferably selected from trifluoromethylsulfinylfluoride, trifluoromethylsulfinylchloride, trifluoromethylsulfinylbromide, trifluoromethylsulfinyliodide, trifluoromethylsulfinic acid, trifluoromethylsulfinic acid anhydride, trifluoromethylsulfinate sodium salt, trifluoromethylsulfinate potassium salt, and mixtures of these.

The sulfinylating agent is more preferably selected from trifluoromethylsulfinic acid, trifluoromethylsulfinic acid anhydride, trifluoromethylsulfinate sodium salt, trifluoromethylsulfinate potassium salt, and mixtures of these.

According to a preferred embodiment of the present invention, trifluoromethylsulfinylfluoride, trifluoromethylsulfinylchloride, trifluoromethylsulfinylbromide, or trifluoromethylsulfinyliodide, more preferably trifluoromethylsulfinylchloride, is used as the sulfinylating agent.

According to a preferred embodiment of the present invention, trifluoromethylsulfinate sodium salt is used as the sulfinylating agent.

According to another preferred embodiment of the present invention, trifluoromethylsulfinate potassium salt is used as the sulfinylating agent.

According to yet another preferred embodiment of the present invention, trifluoromethylsulfinic acid is used as the sulfinylating agent.

According to yet another preferred embodiment of the present invention, trifluoromethylsulfinic acid anhydride is used as the sulfinylating agent.

According to a preferred embodiment of the present invention, a mixture of the trifluoromethylsulfinate sodium and potassium salts, in a mixing ratio of from 0,01 : 99,99 weight % to 50 : 50 weight % is used as the sulfinylating agent.

Preferably, 1.0 to 1.35 molar equivalents, most preferably 1.2 molar equivalents, of the sulfinylating agent relative to 5-amino-1-[2,6-dichloro-4-(trifluoromethyl)phenyl]-1H-pyrazole-3-carbonitrile are used.

In a preferred embodiment, the sulfinylating agent is dried before its use until it is essentially water-free. "Water free" means that the content of water in the solid does not exceed 5 ppm to 100 ppm.

The halogenating agent is selected from thionylchloride, thionylbromide, phosphoroxychloride, oxalylchloride, phosgen, triphosgen ((CCl₃)₂C(=O)), chloroformiates, phosphorpentachloride, phosphortrichloride, trichloromethylchloromethanoat, and xylenesulfonic acid chloride.

According to a preferred embodiment of the present invention, a chlorinating agent is used as the halogenting agent. Preferably, thionylchloride or phosphoroxychloride are used as the chlorinating agent.

According to another preferred embodiment of the present invention, phosphoroxychloride is used as the chlorinating agent.

Most preferably, thionylchloride is used as the chlorinating agent.

Preferably, 1.15 to 1.35 molar equivalents, most preferably about 1.2 molar equivalents, of the halogenating agent relative to 5-amino-1-[2,6-dichloro-4-(trifluoromethyl)phenyl]-1H-pyrazole-3-carbonitrile are used.

We have found that, besides the reaction temperature, the choice of the amine acid complex plays a key role in the sulfinylation of 5-amino-1-[2,6-dichloro-4-(trifluoromethyl)phenyl]-1H-pyrazole-3-carbonitrile. Critical properties that influence the sulfinylating reaction are: steric (bulk) properties, pKs value, solubility, and molecular weight.

The sulfinylation reaction of the present invention is a one-pot synthesis of a two-step reaction. The first step involves the addition of the CF₃S(O)- group to the amino group of the pyrazole ring. In a second step, fipronil is formed via a Thia-Fries rearrangement:

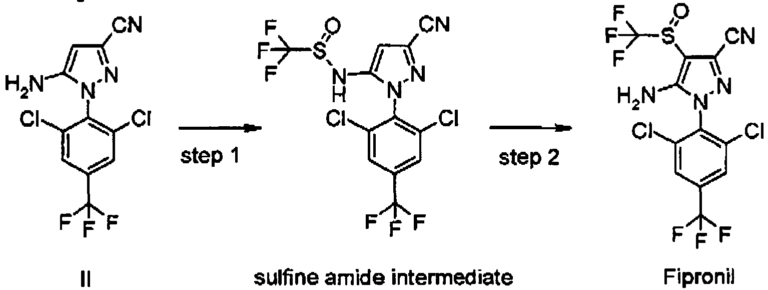

The amine acid complex has two functions in this two-step reaction: (1) when sulfinylates are used as sulfinylating agents, it catalyzes the activation of the sulfinylate with the halogenating agent via intermediate formation of sulfinic acid. For this, catalytical amounts of 0,01 to 1,0 molar equivalents of amine acid complex relative to the pyrazole compound II are needed. (2) It accelerates the Thia-Fries rearrangement and has a significant influence on selectivity. With a view to obtain high yields and high purity, overall amounts of above 1 molar equivalents of amine acid complex relative to the pyrazole compound II are advantageously used for step two.

Preferred are amine acid complexes exhibiting low or essentially no hygroscopicity, as the sulfinylating process of the present invention advantageously is conducted in the essential absence of water (i.e. below 5 to 100 ppm of water).

Preferred amines for use in the present invention are
tertiary amines such as alkyl amines such as trimethylamine, triethylamine, tripropylamine, triisopropylamine, tributylamine, dimethyl ethyl amine, diethyl methylamine, dimethyl n-propyl amine, diisopropylethylamine, DBU (1,8-diazabicyclo[5.4.0]undeo-7-ene), DBN (1,5-diazabicyclo[4.3.0]non-5-ene), methyl morpholine, ethyl morpholine, N,N-dimethylaniline, methyl piperidine, methyl pyrrolidine, or methyldibenzylamine; or aromatic amines such as pyridine, DMAP (dimethylaminopyridine), collidine, lutidine, pyrimidine, pyrazine, or piperazine; or
secondary amines such as alkyl amines such as dimethylamine, diethylamine, dipropylamine, diisopropylamine, dibutylamine, ethylmethylamine, isopropylmethylamine, or isopropylethylamine; or
cyclic secondary amines of the formula NHR¹R² wherein R¹ and R² together with the nitrogen atom to which they are attached form a 3- to 10-membered saturated or partially unsaturated heterocyclic ring system which is unsubstituted or substituted with 1 to 3 C₁-C₈-alkyl or C₁-C₈-haloalkyl groups and which may contain 1 to 3 further heteroatoms selected from oxygen, nitrogen and sulfur.

Preferred amines for use in the present invention are secondary amines.

Preferred secondary amines are alkyl amines such as dimethylamine, diethylamine, dipropylamine, diisopropylamine, dibutylamine, ethylmethylamine, isopropylmethylamine, or isopropylethylamine.

Preferably, the cyclic secondary amines of the amine acid complex are defined by the formula NHR¹R² wherein R¹ and R² together with the nitrogen atom to which they are attached form a 3- to 10-membered saturated or partially unsaturated heterocyclic ring system, preferably a 5- to 6-membered saturated heterocyclic ring system, which is unsubstituted or substituted with 1 to 3 C₁-C₈-alkyl or C₁-C₈-haloalkyl groups, preferably with 1 to 3 C₁-C₃-alkyl or C₁-C₃-haloalkyl groups, most preferably with 1 to 3 C₁-C₃-alkyl groups, most preferably with 1 to 3 methyl groups, and which may contain 1 to 3 further heteroatoms selected from oxygen, nitrogen and sulphur, preferably from oxygen or nitrogen.

Furthermore, preferred secondary amines are cyclic secondary amines such as piperidine, piperidine which is substituted by C₁-C₈-alkyl or C₁-C₈-haloalkyl, preferably C₁-C₂-alkyl or C₁-C₂-haloalkyl, such as 2-methylpiperidine or 4-methylpiperidine, pyrrolidine, imidazolidine, pyrrole, piperazine, or morpholine, preferably morpholine, piperidine, pyrrolidine, pyrrolidine which is substituted by C₁-C₈-alkyl or C₁-C₈-haloalkyl, such as 2-methylpyrrolidine and 3-methylpyrrolidine.

In case secondary amines are used, the respective acid of the amine acid complex preferably is selected from the sulfonic acid derivatives.

Especially preferred amines for use in the present invention are tertiary amines.

Preferred tertiary amines are
alkyl amines such as trimethylamine, triethylamine, tripropylamine, triisopropylamine, dimethyl ethyl amine, diethyl methylamine, dimethyl n-propyl amine, diisopropylethylamine, DBU (1,8-diazabicyclo[5.4.0]undec-7-ene), DBN (1,5-diazabicyclo[4.3.0]non-5-ene), methyl morpholine, ethyl morpholine, N,N-dimethylaniline, methyl piperidine, methyl pyrrolidine, or methyldibenzylamine; or
aromatic amines such as pyridine, DMAP (dimethylaminopyridine), collidine, lutidine, pyrimidine, pyrazine, or piperazine.

In a further preferred embodiment, the tertiary amine is selected from triethylamine, tripropylamine, triisopropylamine, tributylamine, diisopropylethylamine, DBU (1,8-diazabicyclo[5.4.0]undec-7-ene), DBN (1,5-diazabicyclo[4.3.0]non-5-ene), methyl morpholine, ethyl morpholine, N,N-dimethylaniline, methyl piperidine, methyl pyrrolidine, or methyldibenzylamine, DMAP (dimethylaminopyridine), collidine, lutidine, pyrimidine, pyrazine, and piperazine.

In another preferred embodiment, the tertiary amine is selected from trimethylamine, triethylamine, dimethyl ethyl amine, diethyl methylamine, dimethyl n-propyl amine, methyl morpholine, N,N-dimethylaniline, methyl piperidine, methyl pyrrolidine, methyldibenzylamine, and pyridine.

Especially preferred are trimethylamine, triethylamine, dimethyl ethyl amine, dimethyl n-propyl amine, or pyridine. Very preferred are triethylamine and pyridine. In a very preferred embodiment, the amine is triethylamine. Also, in a very preferred embodiment the amine is pyridine. Also, in a very preferred embodiment the amine is trimethylamine. Also, in a very preferred embodiment at least one of the alkyl groups attached to the nitrogen atom of the amine is a methyl group. Also, in a very preferred embodiment the nitrogen atom of the amine group is sp³ hybridized, i.e. it does not form a double bond to a neighbour atom.

Preferred acids of the amine acid complex for use in the present invention are hydrochoric acid, hydrofluoric acid, hydrobromic acid, hydroiodic acid or sulfonic acid derivatives such as aromatic sulfonic acids, e.g. p-toluenesulfonic acid, benzenesulfonic acid, 4-ethyl benzenesulfonic acid, 4-chlorobenzenesulfonic acid, xylene sulfonic acid, 2,3-dimethylbenzene sulfonic acid, 2,4-dimethylbenzene sulfonic acid, 2,5-dimethylbenzene sulfonic acid, 2,6-dimethylbenzene sulfonic acid, 1-napthalenesulfonic acid, 2-napthalenesulfonic acid, mixtures of two or more of the isomers of dimethylbenzene sulfonic acids, or mesitylene sulfonic acid; or alkyl sulfonic acids, e.g. methane sulfonic acid or camphor sulfonic acid; or haloalkylsulfonic acids, e.g. trifluoromethylsulfonic acid. Especially preferred are acids with a pKs-value of below 2.

Most preferred acids are hydrochloric acid and p-toluenesulfonic acid, xylene sulfonic acid, benzene sulfonic acid, methane sulfonic acid, trifluoromethyl sulfonic acid, mesitylene sulfonic acid, especially p-toluenesulfonic acid, xylene sulfonic acid, and benzene sulfonic acid.

In case the amine is a secondary amine, the use of sulfonic acid derivatives is preferred.

From the choice of amine acid complexes as used in the present invention, those with a pKa below 6, preferably 5, and above 10 are preferred.

Preferred amine acid complexes Q are listed in Table 1 which follows.

**Table 1**

| No. | amine | Acid |
|---|---|---|
| Q-1 | N(CH₃)₃ | HF |
| Q-2 | N(CH₃)₃ | HCl |
| Q-3 | N(CH₃)₃ | HBr |
| Q-4 | N(CH₃)₃ | Hl |
| Q-5 | N(CH₃)₃ | p-toluene sulfonic acid |
| Q-6 | N(CH₃)₃ | benzene sulfonic acid |
| Q-7 | N(CH₃)₃ | xylene sulfonic acid |
| Q-8 | N(CH₃)₃ | methane sulfonic acid |
| Q-9 | N(CH₃)₃ | trifluoromethyl sulfonic acid |
| Q-10 | N(CH₃)₃ | mesitylene sulfonic acid |
| Q-11 | N(CH₂CH₃)₃ | HF |
| Q-12 | N(CH₂CH₃)₃ | HCl |
| Q-13 | N(CH₂CH₃)₃ | HBr |
| Q-14 | N(CH₂CH₃)₃ | Hl |
| Q-15 | N(CH₂CH₃)₃ | p-toluene sulfonic acid |
| Q-16 | N(CH₂CH₃)₃ | benzene sulfonic acid |
| Q-17 | N(CH₂CH₃)₃ | xylene sulfonic acid |
| Q-18 | N(CH₂CH₃)₃ | methane sulfonic acid |
| Q-19 | N(CH₂CH₃)₃ | trifluoromethyl sulfonic acid |
| Q-20 | N(CH₂CH₃)₃ | mesitylene sulfonic acid |
| Q-21 | N(CH₂CH₂CH₃)₃ | HF |
| Q-22 | N(CH₂CH₂CH₃)₃ | HCl |
| Q-23 | N(CH₂CH₂CH₃)₃ | HBr |
| Q-24 | N(CH₂CH₂CH₃)₃ | Hl |
| Q-25 | N(CH₂CH₂CH₃)₃ | p-toluene sulfonic acid |
| Q-26 | N(CH₂CH₂CH₃)₃ | benzene sulfonic acid |
| Q-27 | N(CH₂CH₂CH₃)₃ | xylene sulfonic acid |
| Q-28 | N(CH₂CH₂CH₃)₃ | methane sulfonic acid |
| Q-29 | N(CH₂CH₂CH₃)₃ | trifluoromethyl sulfonic acid |
| Q-30 | N(CH₂CH₂CH₃)₃ | mesitylene sulfonic acid |
| Q-31 | N(CH₂CH₂CH₂CH₃)₃ | HF |
| Q-32 | N(CH₂CH₂CH₂CH₃)₃ | HCl |
| Q-33 | N(CH₂CH₂CH₂CH₃)₃ | HBr |
| Q-34 | N(CH₂CH₂CH₂CH₃)₃ | Hl |
| Q-35 | N(CH₂CH₂CH₂CH₃)₃ | p-toluene sulfonic acid |
| Q-36 | N(CH₂CH₂CH₂CH₃)₃ | benzene sulfonic acid |
| Q-37 | N(CH₂CH₂CH₂CH₃)₃ | xylene sulfonic acid |
| Q-38 | N(CH₂CH₂CH₂CH₃)₃ | methane sulfonic acid |
| Q-39 | N(CH₂CH₂CH₂CH₃)₃ | trifluoromethyl sulfonic acid |
| Q-40 | N(CH₂CH₂CH₂CH₃)₃ | mesitylene sulfonic acid |
| Q-41 | N[CH(CH₃)₂]₃ | HF |
| Q-42 | N[CH(CH₃)₂]₃ | HCl |
| Q-43 | N[CH(CHs)₂]₃ | HBr |
| Q-44 | N[CH(CH₃)₂]₃ | Hl |
| Q-45 | N[CH(CH₃)₂]₃ | p-toluene sulfonic acid |
| Q-46 | N[CH(CH₃)₂]₃ | benzene sulfonic acid |
| Q-47 | N[CH(CH₃)₂]₃ | xylene sulfonic acid |
| Q-48 | N[CH(CH₃)₂]₃ | methane sulfonic acid |
| Q-49 | N[CH(CH₃)₂]₃ | trifluoromethyl sulfonic acid |
| Q-50 | N[CH(CH₃)₂]₃ | mesitylene sulfonic acid |
| Q-51 | N(CH₂CH₃)[(CH(CH₃)₂]₂ | HF |
| Q-52 | N(CH₂CH₃)[(CH(CH₃)₂]₂ | HCl |
| Q-53 | N(CH₂CH₃)[(CH(CH₃)₂]₂ | HBr |
| Q-54 | N(CH₂CH₃)[(CH(CH₃)₂]₂ | Hl |
| Q-55 | N(CH₂CH₃)[(CH(CH₃)₂]₂ | p-toluene sulfonic acid |
| Q-56 | N(CH₂CH₃)[(CH(CH₃)₂]₂ | benzene sulfonic acid |
| Q-57 | N(CH₂CH₃)[(CH(CH₃)₂]₂ | xylene sulfonic acid |
| Q-58 | N(CH₂CH₃)[(CH(CH₃)₂]₂ | methane sulfonic acid |
| Q-59 | N(CH₂CH₃)[(CH(CH₃)₂]₂ | trifluoromethyl sulfonic acid |
| Q-60 | N(CH₂CH₃)[(CH(CH₃)₂]₂ | mesitylene sulfonic acid |
| Q-61 | DBU | HF |
| Q-62 | DBU | HCl |
| Q-63 | DBU | HBr |
| Q-64 | DBU | Hl |
| Q-65 | DBU | p-toluene sulfonic acid |
| Q-66 | DBU | benzene sulfonic acid |
| Q-67 | DBU | xylene sulfonic acid |
| Q-68 | DBU | methane sulfonic acid |
| Q-69 | DBU | trifluoromethyl sulfonic acid |
| Q-70 | DBU | mesitylene sulfonic acid |
| Q-71 | DBN | HF |
| Q-72 | DBN | HCl |
| Q-73 | DBN | HBr |
| Q-74 | DBN | Hl |
| Q-75 | DBN | p-toluene sulfonic acid |
| Q-76 | DBN | benzene sulfonic acid |
| Q-77 | DBN | xylene sulfonic acid |
| Q-78 | DBN | methane sulfonic acid |
| Q-79 | DBN | trifluoromethyl sulfonic acid |
| Q-80 | DBN | mesitylene sulfonic acid |
| Q-81 | methyl morpholine | HF |
| Q-82 | methyl morpholine | HCl |
| Q-83 | methyl morpholine | HBr |
| Q-84 | methyl morpholine | Hl |
| Q-85 | methyl morpholine | p-toluene sulfonic acid |
| Q-86 | methyl morpholine | benzene sulfonic acid |
| Q-87 | methyl morpholine | xylene sulfonic acid |
| Q-88 | methyl morpholine | methane sulfonic acid |
| Q-89 | methyl morpholine | trifluoromethyl sulfonic acid |
| Q-90 | methyl morpholine | mesitylene sulfonic acid |
| Q-91 | ethyl morpholine | HF |
| Q-92 | ethyl morpholine | HCl |
| Q-93 | ethyl morpholine | HBr |
| Q-94 | ethyl morpholine | Hl |
| Q-95 | ethyl morpholine | p-toluene sulfonic acid |
| Q-96 | ethyl morpholine | benzene sulfonic acid |
| Q-97 | ethyl morpholine | xylene sulfonic acid |
| Q-98 | ethyl morpholine | methane sulfonic acid |
| Q-99 | ethyl morpholine | trifluoromethyl sulfonic acid |
| Q-100 | ethyl morpholine | mesitylene sulfonic acid |
| Q-101 | N,N-dimethylaniline | HF |
| Q-102 | N,N-dimethylaniline | HCl |
| Q-103 | N,N-dimethylaniline | HBr |
| Q-104 | N,N-dimethylaniline | Hl |
| Q-105 | N,N-dimethylaniline | p-toluene sulfonic acid |
| Q-106 | N,N-dimethylaniline | benzene sulfonic acid |
| Q-107 | N,N-dimethylaniline | xylene sulfonic acid |
| Q-108 | N,N-dimethylaniline | methane sulfonic acid |
| Q-109 | N,N-dimethylaniline | trifluoromethyl sulfonic acid |
| Q-110 | N,N-dimethylaniline | mesitylene sulfonic acid |
| Q-111 | methyl piperidine | HF |
| Q-112 | methyl piperidine | HCl |
| Q-113 | methyl piperidine | HBr |
| Q-114 | methyl piperidine | Hl |
| Q-115 | methyl piperidine | p-toluene sulfonic acid |
| Q-116 | methyl piperidine | benzene sulfonic acid |
| Q-117 | methyl piperidine | xylene sulfonic acid |
| Q-118 | methyl piperidine | methane sulfonic acid |
| Q-119 | methyl piperidine | trifluoromethyl sulfonic acid |
| Q-120 | methyl piperidine | mesitylene sulfonic acid |
| Q-121 | methyl pyrrolidine | HF |
| Q-122 | methyl pyrrolidine | HCl |
| Q-123 | methyl pyrrolidine | HBr |
| Q-124 | methyl pyrrolidine | Hl |
| Q-125 | methyl pyrrolidine | p-toluene sulfonic acid |
| Q-126 | methyl pyrrolidine | benzene sulfonic acid |
| Q-127 | methyl pyrrolidine | xylene sulfonic acid |
| Q-128 | methyl pyrrolidine | methane sulfonic acid |
| Q-129 | methyl pyrrolidine | trifluoromethyl sulfonic acid |
| Q-130 | methyl pyrrolidine | mesitylene sulfonic acid |
| Q-131 | methyl dibenzyl amine | HF |
| Q-132 | methyl dibenzyl amine | HCl |
| Q-133 | methyl dibenzyl amine | HBr |
| Q-134 | methyl dibenzyl amine | Hl |
| Q-135 | methyl dibenzyl amine | p-toluene sulfonic acid |
| Q-136 | methyl dibenzyl amine | benzene sulfonic acid |
| Q-137 | methyl dibenzyl amine | xylene sulfonic acid |
| Q-138 | methyl dibenzyl amine | methane sulfonic acid |
| Q-139 | methyl dibenzyl amine | trifluoromethyl sulfonic acid |
| Q-140 | methyl dibenzyl amine | mesitylene sulfonic acid |
| Q-141 | pyridine | HF |
| Q-142 | pyridine | HCl |
| Q-143 | pyridine | HBr |
| Q-144 | pyridine | Hl |
| Q-145 | pyridine | p-toluene sulfonic acid |
| Q-146 | pyridine | benzene sulfonic acid |
| Q-147 | pyridine | xylene sulfonic acid |
| Q-148 | pyridine | methane sulfonic acid |
| Q-149 | pyridine | trifluoromethyl sulfonic acid |
| Q-150 | pyridine | mesitylene sulfonic acid |
| Q-151 | DMAP | HF |
| Q-152 | DMAP | HCl |
| Q-153 | DMAP | HBr |
| Q-154 | DMAP | Hl |
| Q-155 | DMAP | p-toluene sulfonic acid |
| Q-156 | DMAP | benzene sulfonic acid |
| Q-157 | DMAP | xylene sulfonic acid |
| Q-158 | DMAP | methane sulfonic acid |
| Q-159 | DMAP | trifluoromethyl sulfonic acid |
| Q-160 | DMAP | mesitylene sulfonic acid |
| Q-161 | collidine | HF |
| Q-162 | collidine | HCl |
| Q-163 | collidine | HBr |
| Q-164 | collidine | Hl |
| Q-165 | collidine | p-toluene sulfonic acid |
| Q-166 | collidine | benzene sulfonic acid |
| Q-167 | collidine | xylene sulfonic acid |
| Q-168 | collidine | methane sulfonic acid |
| Q-169 | collidine | trifluoromethyl sulfonic acid |
| Q-170 | collidine | mesitylene sulfonic acid |
| Q-171 | lutidine | HF |
| Q-172 | lutidine | HCl |
| Q-173 | lutidine | HBr |
| Q-174 | lutidine | Hl |
| Q-175 | lutidine | p-toluene sulfonic acid |
| Q-176 | lutidine | benzene sulfonic acid |
| Q-177 | lutidine | xylene sulfonic acid |
| Q-178 | lutidine | methane sulfonic acid |
| Q-179 | lutidine | trifluoromethyl sulfonic acid |
| Q-180 | lutidine | mesitylene sulfonic acid |
| Q-181 | pyrimidine | HF |
| Q-182 | pyrimidine | HCl |
| Q-183 | pyrimidine | HBr |
| Q-184 | pyrimidine | Hl |
| Q-185 | pyrimidine | p-toluene sulfonic acid |
| Q-186 | pyrimidine | benzene sulfonic acid |
| Q-187 | pyrimidine | xylene sulfonic acid |
| Q-188 | pyrimidine | methane sulfonic acid |
| Q-189 | pyrimidine | trifluoromethyl sulfonic acid |
| Q-190 | pyrimidine | mesitylene sulfonic acid |
| Q-191 | pyrazine | HF |
| Q-192 | pyrazine | HCl |
| Q-193 | pyrazine | HBr |
| Q-194 | pyrazine | Hl |
| Q-195 | pyrazine | p-toluene sulfonic acid |
| Q-196 | pyrazine | benzene sulfonic acid |
| Q-197 | pyrazine | xylene sulfonic acid |
| Q-198 | pyrazine | methane sulfonic acid |
| Q-199 | pyrazine | trifluoromethyl sulfonic acid |
| Q-200 | pyrazine | mesitylene sulfonic acid |
| Q-201 | piperazine | HF |
| Q-202 | piperazine | HCl |
| Q-203 | piperazine | HBr |
| Q-204 | piperazine | Hl |
| Q-205 | piperazine | p-toluene sulfonic acid |
| Q-206 | piperazine | benzene sulfonic acid |
| Q-207 | Piperazine | xylene sulfonic acid |
| Q-208 | Piperazine | methane sulfonic acid |
| Q-209 | Piperazine | trifluoromethyl sulfonic acid |
| Q-210 | Piperazine | mesitylene sulfonic acid |
| Q-211 | N(CH₃)₂CH₂CH₃ | HF |
| Q-212 | N(CH₃)₂CH₂CH₃ | HCl |
| Q-213 | N(CH₃)₂CH₂CH₃ | HBr |
| Q-214 | N(CH₃)₂CH₂CH₃ | Hl |
| Q-215 | N(CH₃)₂CH₂CH₃ | p-toluene sulfonic acid |
| Q-216 | N(CH₃)₂CH₂CH₃ | benzene sulfonic acid |
| Q-217 | N(CH₃)₂CH₂CH₃ | xylene sulfonic acid |
| Q-218 | N(CH₃)₂CH₂CH₃ | methane sulfonic acid |
| Q-219 | N(CH₃)₂CH₂CH₃ | trifluoromethyl sulfonic acid |
| Q-220 | N(CH₃)₂CH₂CH₃ | mesitylene sulfonic acid |
| Q-221 | N(CH₃)₂CH₂CH₂CH₃ | HF |
| Q-222 | N(CH₃)₂CH₂CH₂CH₃ | HCl |
| Q-223 | N(CH₃)₂CH₂CH₂CH₃ | HBr |
| Q-224 | N(CH₃)₂CH₂CH₂CH₃ | Hl |
| Q-225 | N(CH₃)₂CH₂CH₂CH₃ | p-toluene sulfonic acid |
| Q-226 | N(CH₃)₂CH₂CH₂CH₃ | benzene sulfonic acid |
| Q-227 | N(CH₃)₂CH₂CH₂CH₃ | xylene sulfonic acid |
| Q-228 | N(CH₃)₂CH₂CH₂CH₃ | methane sulfonic acid |
| Q-229 | N(CH₃)₂CH₂CH₂CH₃ | trifluoromethyl sulfonic acid |
| Q-230 | N(CH₃)₂CH₂CH₂CH₃ | mesitylene sulfonic acid |
| Q-231 | NCH₃(CH₂CH₃)₂ | HF |
| Q-232 | NCH₃(CH₂CH₃)₂ | HCl |
| Q-233 | NCH₃(CH₂CH₃)₂ | HBr |
| Q-234 | NCH₃(CH₂CH₃)₂ | Hl |
| Q-235 | NCH₃(CH₂CH₃)₂ | p-toluene sulfonic acid |
| Q-236 | NCH₃(CH₂CH₃)₂ | benzene sulfonic acid |
| Q-237 | NCH₃(CH₂CH₃)₂ | xylene sulfonic acid |
| Q-238 | NCH₃(CH₂CH₃)₂ | methane sulfonic acid |
| Q-239 | NCH₃(CH₂CH₃)₂ | trifluoromethyl sulfonic acid |
| Q-240 | NCH₃(CH₂CH₃)₂ | mesitylene sulfonic acid |
| Q-241 | NH(CH₃)₂ | p-toluene sulfonic acid |
| Q-242 | NH(CH₃)₂ | benzene sulfonic acid |
| Q-243 | NH(CH₃)₂ | xylene sulfonic acid |
| Q-244 | NH(CH₃)₂ | methane sulfonic acid |
| Q-245 | NH(CH₃)₂ | trifluoromethyl sulfonic acid |
| Q-246 | NH(CH₃)₂ | mesitylene sulfonic acid |
| Q-247 | NH(CH₂CH₃)₂ | p-toluene sulfonic acid |
| Q-248 | NH(CH₂CH₃)₂ | benzene sulfonic acid |
| Q-249 | NH(CH₂CH₃)₂ | xylene sulfonic acid |
| Q-250 | NH(CH₂CH₃)₂ | methane sulfonic acid |
| Q-251 | NH(CH₂CH₃)₂ | trifluoromethyl sulfonic acid |
| Q-252 | NH(CH₂CH₃)₂ | mesitylene sulfonic acid |
| Q-253 | NH(CH₂CH₂CH₃)₂ | p-toluene sulfonic acid |
| Q-254 | NH(CH₂CH₂CH₃)₂ | benzene sulfonic acid |
| Q-255 | NH(CH₂CH₂CH₃)₂ | xylene sulfonic acid |
| Q-256 | NH(CH₂CH₂CH₃)₂ | methane sulfonic acid |
| Q-257 | NH(CH₂CH₂CH₃)₂ | trifluoromethyl sulfonic acid |
| Q-258 | NH(CH₂CH₂CH₃)₂ | mesitylene sulfonic acid |
| Q-259 | NH(CH(CH₃)₂)₂ | p-toluene sulfonic acid |
| Q-260 | NH(CH(CH₃)₂)₂ | benzene sulfonic acid |
| Q-261 | NH(CH(CH₃)₂)₂ | xylene sulfonic acid |
| Q-262 | NH(CH(CH₃)₂)₂ | methane sulfonic acid |
| Q-263 | NH(CH(CH₃)₂)₂ | trifluoromethyl sulfonic acid |
| Q-264 | NH(CH(CH₃)₂)₂ | mesitylene sulfonic acid |
| Q-265 | NHCH₃(CH(CH₃)₂) | p-toluene sulfonic acid |
| Q-266 | NHCH₃(CH(CH₃)₂) | benzene sulfonic acid |
| Q-267 | NHCH₃(CH(CH₃)₂) | xylene sulfonic acid |
| Q-268 | NHCH₃(CH(CH₃)₂) | methane sulfonic acid |
| Q-269 | NHCH₃(CH(CH₃)₂) | trifluoromethyl sulfonic acid |
| Q-270 | NHCH₃(CH(CH₃)₂) | mesitylene sulfonic acid |
| Q-271 | NHCH₃(CH₂CH₃) | p-toluene sulfonic acid |
| Q-272 | NHCH₃(CH₂CH₃) | benzene sulfonic acid |
| Q-273 | NHCH₃(CH₂CH₃) | xylene sulfonic acid |
| Q-274 | NHCH₃(CH₂CH₃) | methane sulfonic acid |
| Q-275 | NHCH₃(CH₂CH₃) | trifluoromethyl sulfonic acid |
| Q-276 | NHCH₃(CH₂CH₃) | mesitylene sulfonic acid |
| Q-277 | Morpholine | p-toluene sulfonic acid |
| Q-278 | Morpholine | benzene sulfonic acid |
| Q-279 | Morpholine | xylene sulfonic acid |
| Q-280 | Morpholine | methane sulfonic acid |
| Q-281 | Morpholine | trifluoromethyl sulfonic acid |
| Q-282 | Morpholine | mesitylene sulfonic acid |
| Q-283 | Piperidine | p-toluene sulfonic acid |
| Q-284 | Piperidine | benzene sulfonic acid |
| Q-285 | Piperidine | xylene sulfonic acid |
| Q-286 | Piperidine | methane sulfonic acid |
| Q-287 | Piperidine | trifluoromethyl sulfonic acid |
| Q-288 | Piperidine | mesitylene sulfonic acid |
| Q-289 | 4-methylpiperidine | p-toluene sulfonic acid |
| Q-290 | 4-methylpiperidine | benzene sulfonic acid |
| Q-291 | 4-methylpiperidine | xylene sulfonic acid |
| Q-292 | 4-methylpiperidine | methane sulfonic acid |
| Q-293 | 4-methylpiperidine | trifluoromethyl sulfonic acid |
| Q-294 | 4-methylpiperidine | mesitylene sulfonic acid |
| Q-295 | Pyrrolidine | p-toluene sulfonic acid |
| Q-296 | Pyrrolidine | benzene sulfonic acid |
| Q-297 | Pyrrolidine | xylene sulfonic acid |
| Q-298 | Pyrrolidine | methane sulfonic acid |
| Q-299 | Pyrrolidine | trifluoromethyl sulfonic acid |
| Q-300 | Pyrrolidine | mesitylene sulfonic acid |
| Q-301 | Imidazole | p-toluene sulfonic acid |
| Q-302 | Imidazole | benzene sulfonic acid |
| Q-303 | Imidazole | xylene sulfonic acid |
| Q-304 | Imidazole | methane sulfonic acid |
| Q-305 | Imidazole | trifluoromethyl sulfonic acid |
| Q-306 | Imidazole | trifluoromethyl sulfonic acid |
| Q-307 | Imidazole | mesitylene sulfonic acid |
| Q-308 | Pyrrole | p-toluene sulfonic acid |
| Q-309 | Pyrrole | benzene sulfonic acid |
| Q-310 | Pyrrole | xylene sulfonic acid |
| Q-311 | Pyrrole | methane sulfonic acid |
| Q-312 | Pyrrole | trifluoromethyl sulfonic acid |
| Q-313 | Pyrrole | mesitylene sulfonic acid |
| Q-314 | Piperazine | p-toluene sulfonic acid |
| Q-315 | Piperazine | benzene sulfonic acid |
| Q-316 | Piperazine | xylene sulfonic acid |
| Q-317 | Piperazine | methane sulfonic acid |
| Q-318 | Piperazine | trifluoromethyl sulfonic acid |
| Q-319 | Piperazine | mesitylene sulfonic acid |
| Q-320 | 4-methylpiperidine | p-toluene sulfonic acid |
| Q-321 | 4-methylpiperidine | benzene sulfonic acid |
| Q-322 | 4-methylpiperidine | xylene sulfonic acid |
| Q-323 | 4-methylpiperidine | methane sulfonic acid |
| Q-324 | 4-methylpiperidine | trifluoromethyl sulfonic acid |
| Q-325 | 4-methylpiperidine | mesitylene sulfonic acid |
| Q-326 | 2-methylpyrrolidine | p-toluene sulfonic acid |
| Q-327 | 2-methylpyrrolidine | benzene sulfonic acid |
| Q-328 | 2-methylpyrrolidine | xylene sulfonic acid |
| Q-329 | 2-methylpyrrolidine | methane sulfonic acid |
| Q-330 | 2-methylpyrrolidine | trifluoromethyl sulfonic acid |
| Q-331 | 2-methylpyrrolidine | mesitylene sulfonic acid |

When the amine is a tertiary amine, amine acid complexes of Table 1 wherein the acid is hydrochloric acid are preferred.

Also, those amine acid complexes of Table 1 wherein the amine is a tertiary amine and the acid is p-toluene sulfonic acid, benzene sulfonic acid, or xylene sulfonic acid are preferred.

Amine acid complexes of Table 1 wherein the amine is a tertiary amine Q-2, Q-5, Q-12, Q-17, Q-22, Q-32, Q-42, Q-52, Q-142, Q-145, Q-147, Q-162, Q-165, Q-172, Q-175, Q-212, Q-215, Q-222, Q-225, Q-232, or Q-235 are preferred.

Even more preferred are amine acid complexes of Table 1 wherein the amine is a tertiary amine Q-2, Q-5, Q-6, Q-7, Q-12, Q-82, Q-85, Q-86, Q-87, Q-102, Q-105, Q-106, Q-107, Q-112, Q-115, Q-116, Q-117, Q-122, Q-125, Q-126, Q-127, Q-132, Q-135, Q-136. Q-137, Q-142, Q-145, Q-146, Q-147, Q-212, Q-215, Q-216, Q-217, Q-222, Q-225, Q-226, Q-227, Q-232, Q-235, Q-236, or Q-237.

Most preferred are amine acid complexes of Table 1 wherein the amine is a tertiary amine Q-2, Q-5, Q-6, Q-7, Q-12, Q-142, Q-145, Q-146, Q-147, Q-212, Q-215, Q-216, Q-217, Q-222, Q-225, Q-226, Q-227, Q-232, Q-235, Q-236, or Q-237.

Amine acid complexes of Table 1 wherein the amine is an alkyl amine Q-241, Q-242, Q-243, Q-247 or Q-249 are preferred.

Amine acid complexes of Table 1 wherein the amine is a cyclic secondary amine Q-277, Q-278, Q279, Q-283, Q-284, Q285, Q-295, Q-296, Q-297, Q-314, Q-315, Q-316, Q-320, Q-321, Q-323, Q-326, Q-327, or Q-328 are preferred.

With regard to their use in the inventive process, the combinations of sulfinylating agent and amine acid complex given in the tables below are especially preferred.

### Table 2

Trifluoromethylsulfinate sodium salt is used as the sulfinylating agent, and the amine acid complex in each case is a row of table 1.

### Table 3

Trifluoromethylsulfinate potassium salt is used as the sulfinylating agent, and the amine acid complex in each case is a row of table 1.

### Table 4

Trifluoromethylsulfinic acid is used as the sulfinylating agent, and the amine acid complex in each case is a row of table 1.

### Table 5

Trifluoromethylsulfinic acid anhydride is used as the sulfinylating agent, and the amine acid complex in each case is a row of table 1.

### Table 6

A mixture of trifluoromethylsulfinate sodium and potassium salt in a mixing ratio of from 0,01 : 99,99 weight % to 50 : 50 weight % is used as the sulfinylating agent, and the amine acid complex in each case is a row of table 1.

### Table 7

Trifluoromethylsulfinylfluoride is used as the sulfinylating agent, and the amine acid complex in each case is a row of table 1.

### Table 8

Trifluoromethylsulfinylchloride is used as the sulfinylating agent, and the amine acid complex in each case is a row of table 1.

### Table 9

Trifluoromethylsulfinylbromide is used as the sulfinylating agent, and the amine acid complex in each case is a row of table 1.

### Table 10

Trifluoromethylsulfinyliodide is used as the sulfinylating agent, and the amine acid complex in each case is a row of table 1.

Also, in a further embodiment of the present invention, a Lewis acid such as AlCl₃, FeCl₃, CaCl₂, ZnCl₂, BF₃, TiCl₄, or ZrCl₄ can be used in exchange for the protonic acids cited above.

It can be advantageous to add the amine acid complex in more than one, such as in two portions, e.g. one portion for step 1 and one portion after the addition of the pyrazole of formula II.

It can be advantageous to use two different amine acid complexes during the course of the reaction. For example, the first amine acid complex can be added in step 1 in amounts of 0,2 to 1 molar equivalents relative to pyrazole II, catalyzing the activation of the sulfinylate with the halogenating agent. After addition of the pyrazole of formula II, in step 2, the Thia-Fries rearrangement, a second amine acid complex different from the first one is added, in amounts of 0,2 to 1 molar equivalents relative to pyrazole II.

Preferably, 1.4 to 2.2 molar equivalents, most preferably 1.5 to 1.8 molar equivalents, of the amine acid complex according to the present invention relative to 5-amino-1-[2,6-dichloro-4-(trifluoromethyl)phenyl]-1H-pyrazole-3-carbonitrile are used.

When the sulfinylating agent is trifluoromethylsulfinic acid or a mixture containing trifluoromethylsulfinic acid, the molar amount of amine acid complex which is molar equivalent to the molar amount of trifluoromethylsulfinic acid is preferably generated in situ by addition of amine, and the remaining molar amount necessary to obtain the required 1.4 to 2.2 molar equivalents is added as amine acid complex.

In a preferred embodiment, the amine acid complex is dried before its use until it is essentially water-free. "Water free" means that the content of water in the solid does not exceed 5 ppm to 100 ppm.

Further additives can advantageously be added to the reaction mixture, such as potassium fluoride, pentafluorophenol, dimethylformamide, or 2,4-dinitrophenol. These additives are preferably added to the reaction mixture or solution or suspension of starting materials, respectively, before or at the reaction start. Most preferably, the additives are added at a low temperature of 5°C to 10°C.

In a preferred embodiment, 0.1 to 1.5 molar equivalents of potassium fluoride relative to 5-amino-1-[2,6-dichloro-4-(trifluoromethyl)phenyl]-1H-pyrazole-3-carbonitrile are added to the reaction mixture or solution or suspension of starting materials, respectively, at 5°C to 10°C at or before the reaction start.

Is is advantageous to add pentafluorophenol, dimethylformamide, or 2,4-dinitrophenol in catalytic amounts or in 0.10 molar equivalents relative to 5-amino-1-[2,6-dichloro-4-(trifluoromethyl)phenyl]-1H-pyrazole-3-carbonitrile.

In a preferred embodiment, the additive is dried before its use until it is essentially water-free. "Water free" means that the content of water in the solid does not exceed 5 ppm to 100 ppm.

The reaction can be conducted in an inert organic solvent, preferably selected from
- aliphatic, alicyclic or aromatic, optionally halogenated hydrocarbons such as aromatic organic hydrocarbons, e.g. toluene, xylene, trifluoromethylbenzene, benzene, nitrobenzene, monochlorobenzene, dichlorobenzene, and ethylbenzene, preferably toluene, ethylbenzene and xylene, most preferably toluene; or aliphatic or alicyclic, optionally halogenated hydrocarbons such as hexane, cyclohexane, benzine, 1,2 dichloroethane, dichloromethane, trichloromethane (chloroform), tetrachlorocarbon, preferably 1,2 dichloroethane, dichloromethane, trichloromethane; and
- ethers, e.g. diethylether, dioxane, tetrahydrofuran, 2-methyl-tetrahydrofuran or ethylen glycol dimethyl- or diethylether; and
- ketons, e.g. acetone or butanon ; and
- nitriles, e.g. acetonitrile or propionitrile ; and
- amides, e.g. dimethylformamid, DMI (1,3-dimethyl-2-imidazolidinon), dimethylacetamid, N-methylformanilid, N-methylpyrrolidon or hexamethylphosphoric acid triamide; and
- sulfoxides, e.g. dimethylsulfoxide,
or mixtures thereof.

In a preferred embodiment, solvents which are essentially water free are used. "Water free" means that the content of water in the solvent does not exceed 5 ppm to 100 ppm. The most preferred solvent is water free toluene.

The reaction is carried out under an inert gas atmosphere, such as under an argon or a nitrogen atmosphere.

In a preferred embodiment, a total of 3.0 to 8.0 molar equivalents, more preferably 4.0 to 7.5 molar equivalents, and most preferably 4.5 to 6.5 molar equivalents of the solvent relative to 5-amino-1-[2,6-dichloro-4-(trifluoromethyl)phenyl]-1H-pyrazole-3-carbonitrile are used. This relatively high concentration of starting materials maximizes the conversion to the sulfinamid intermediate.

In cases where the starting materials are dissolved and/or suspended, respectively, separately before their combination, about 25% to 40 % of the solvent are employed for dissolving and/ or suspending the 5-amino-1-[2,6-dichloro-4-(trifluoromethyl)phenyl]-1H-pyrazole-3-carbonitrile.

Generally, the sequence of addition of the starting materials 5-amino-1-[2,6-dichloro-4-(trifluoromethyl)phenyl]-1H-pyrazole-3-carbonitrile, amine acid complex, the sulfinylating agent and the halogenating agent generally can be freely chosen.

Preferably, the respective starting materials are dissolved or suspended, respectively, in the reaction solvent before addition to the reaction mixture.

The halogenating agent is preferably not added to 5-amino-1-[2,6-dichloro-4-(trifluoromethyl)phenyl]-1H-pyrazole-3-carbonitrile in the absence of the amine acid complex or the sulfinylating agent in the reaction mixture. In a preferred embodiment, the halogenating agent is dissolved in the solvent and added to a reaction mixture containing 5-amino-1-[2,6-dichloro-4-(trifluoromethyl)phenyl]-1H-pyrazole-3-carbonitrile, amine acid complex and the sulfinylating agent, all dissolved or suspended, respectively, in the solvent.

In a preferred embodiment, the halogenating agent and the amine acid complex are mixed with the solvent and the dissolved or suspended sulfinylating agent is added to the reaction mixture before 5-amino-1-[2,6-dichloro-4-(trifluoromethyl)phenyl]-1H-pyrazole-3-carbonitrile is added.

In a preferred embodiment, 5-amino-1-[2,6-dichloro-4-(trifluoromethyl)phenyl]-1H-pyrazole-3-carbonitrile (II) is combined with a mixture containing the sulfinylating agent, the amine acid complex and the halogenating agent. In this case, it can be advantageous to include a first portion (equaling about 1 molar equivalent relative to compound II) of halogenating agent in the mixture containing the sulfinylating agent, the amine acid complex and the halogenating agent and then to add the second portion (equaling about 0,1 to 0,2 molar equivalents relative to compound II) after addition of the compound II and stirring for approximately 30 to 60 minutes and shortly before rising the temperature to 30 to 39°C.

When the sulfinylating agent is trifluoromethylsulfinic acid, it is preferred to simultaneously add the trifluoromethylsulfinic acid and the halogenating agent to a solution or suspension of the amine acid complex, followed by addition of a solution of 5-amino-1-[2,6-dichloro-4-(trifluoromethyl)phenyl]-1H-pyrazole-3-carbonitrile to the reaction mixture.

In another preferred embodiment, a dissolved or suspended mixture of the sulfinylating agent, the amine acid complex and the halogenating agent in the solvent (preferably toluene) is cooled to about 3°C to 10°C, and a solution of 5-amino-1-[2,6-dichloro-4-(trifluoromethyl)phenyl]-1H-pyrazole-3-carbonitrile in the solvent (preferably toluene) which as been heated to about 90°C to 110°C is combined with the cooled mixture. The volume ratios of the solvents are chosen in a way to ensure that the reaction temperature does not exceed 39°C.

In a preferred embodiment, after combination of 5-amino-1-[2,6-dichloro-4-(trifluoromethyl)phenyl]-1H-pyrazole-3-carbonitrile, the sulfinylating agent, the amine acid complex, and the chlorinating agent, the temperature is raised to 30°C to 39°C, preferably not above 35°C, within 5 to 60 minutes.

It is also preferred to hold the reaction temperature initially at -20°C to 10°C for 5 to 60 minutes, preferably 20 to 40 minutes, followed by rising the temperature to 30°C to 55 °C at a rate of 5°C / min to 45°C / min. Preferably, in order to obtain high purity products, the reaction mixture is rised to a temperature not above 35°C. When the sulfinylating agent is or contains CF₃S(O)OH, the initial reaction temperature preferably is -20°C to 5°C, in case of trifluoromethylsulfinate alkaline or alkaline earth metal salts, the initial reaction temperature preferably is -5°C to 10°C.

Typically, the reaction time will be about 5 to 15 hours, preferably 10 to 15 hours.

In a further preferred embodiment, the reaction is carried out in a pressure vessel at a pressure of 1, 013 bar (1 atm) to about 4 bar.

After completion of the reaction, fipronil can be isolated by employing conventional methods such as quenching the reaction with hydrogen carbonates, such as NaHCO₃, carbonates such as NaCO₃, or hydroxides, such as NaOH, extracting fipronil with an unpolar organic solvent such as ethylacetate or methyl-tert.-butylether, washing the extract, e.g. with hydrogen carbonates such as NaHCO₃, concentrating the extract, e.g. in vacuo, crystallization of fipronil, and the like. The isolated fipronil can be purified by a technique such as chromatography, recrystallization and the like, if necessary.

The crystallization of the final product 5-amino-1-[2,6-dichloro-4-(trifluoromethyl)phenyl]-4-(trifluoromethylsulfinyl)-pyrazole-3-carbonitrile is typically conducted from a solution in a nonpolar, inert, preferably aromatic solvent with nonreactive substituents such as chloro, fluoro, cyano, nitro, C₁-C₈-alkyl, or C₁-C₈-haloalkyl, particularly from a solution in benzene, ethylbenzene, monochlorobenzene, monofluorobenzene, 1,2-dichlorobenzene, 1,3-dichlorobenzene, 1,4-dichlorobenzene, toluene, o-xylene, m-xylene, p-xylene, styrene, i-propyl benzene, n-propyl benzene, 2-chlorotoluene, 3-chlorotoluene, 4-chlorotoluene, tert.-butyl benzene, sec.-butyl benzene, iso-butyl benzene, n-butyl benzene, 1,3-diisopropylbenzene, 1,4-diisopropylbenzene, 2-nitrotoluene, 3-nitrotoluene, 4-nitrotoluene, nitrobenzene, benzonitrile, mesitylene, trifluoromethyl benzene, 1,2-dichloroethane, acetonitrile, dimethylsulfoxide, tetrahydrofuran, acetone, alcohols such as methanol, ethanol, n-propanol, isopropanol, n-butanol, isobutanol, 2-butanol, or tert-butanol, or mixtures thereof, preferably from a solution in monochlorobenzene, dichlorobenzene, ethylbenzene or toluene.

It can be advantageous to add about 1 to 30 percent of a polar solvent such as ketons, amides, alcohols, esters or ethers, preferably esters, ketons or ethers, such as acetone methyl ethyl ketone, pentan-2-one, diethylketone, 4-methyl-2-pentanone, 3-methylbutan-2-one,
tert-butyl-methyl-ketone, cyclohexanone, methylacetate, ethylacetate, isopropylacetate, N-butylacetate, isobutylacetate, diethylcarbonate, 2-butoxyethylacetate, dimethylformamide, dimethylacetamide, dimethylsulfoxide, nitromethane, nitroethane, water, ethanol, methanol, propan-1-ol, propan-2-ol, butan-1-ol, butan-2-ol, tert-butanol, 2-methyl-propan-1-ol, 2-methyl-propan-2-ol, pentan-3-ol, 2-methyl butan-1-ol, 3-methyl butan-1-ol, 1,2-ethanediol, 1,3-propandiol, 1,2-propandiol, cyclohexanol, dioxane, tetrahydrofurane, diethylether, methyl tert.-butyl ether, 2-methyl tetrahydrofuran, acetonitrile, propionitrile, or mixtures thereof.

In another embodiment, fipronil is crystallized from water, optionally with the addition of about 1 to 30 percent of a polar organic solvent.

Preferably, the crystallization is done from monochlorobenzene.

Preferably, the crystallization is done from dichlorobenzene.

Preferably, the crystallization is done from ethylbenzene.

Preferably, the crystallization is done from toluene.

Purification of the crude product can also be achieved via filtration over charcoal or silica or washing with water.

When obtained according to the inventive process, the obtained product fipronil in the crude reaction mixture before crystallization contains less than 3,0 weight %, calculated without solvent, of compound F, a typical, biologically active side product of fipronil syntheses.

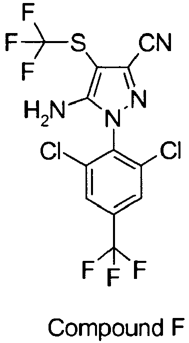

After purification of the crude product by suitable methods such as washing and (re-) crystallizing, fipronil obtained by the inventive process contains less than 1.0 weight% of compound F.

Besides, the obtained product fipronil contains less than 10 ppm of the compound D which is a common side product of the currently large scale industrial process as described e.g. in WO 01/30760, even after purification. Fipronil when prepared by the inventive process in an inert atmosphere contains less than 200 ppm of compounds containing sulfur in its oxidation state (IV). It also is free of compound E which typically can appear as side product of the current industrial process.

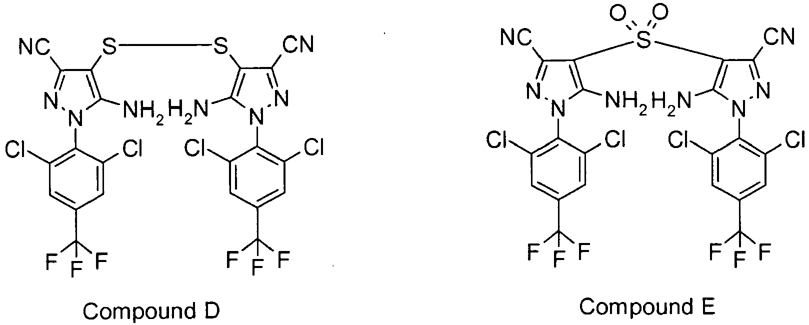

Furthermore, the obtained product fipronil is also free of trifluoroacetic acid, which is a reagent used in the current industrial process.

Moreover, when a chlorinating agent is used as the halogenating agent, the obtained fipronil product is practically free of bromine, meaning that is does not contain more than 5 to 20 ppm of bromine.

### Examples

HPLCs were taken on a Hewlett Packard HP 1200, Chemstation, equipped with a J'Sphere ODS-H80, 4 µm, 4.6 x 250 mm (YMC) column, eluent A: 90 wt.-% water + 10 wt.-% acetonitrile, eluent B: 10 wt.-% water + 90 wt.-% acetonitrile, flow: 0.85 ml/min, detection: 235 nm, gradient:

| | | | | | |
|---|---|---|---|---|---|
| time [min] | 0 | 2 | 17 | 25 | 35 |
| A [%] | 60 | 60 | 25 | 0 | 0 |
| B [%] | 40 | 40 | 75 | 100 | 100. |

Yields given below are in mol percent of the obtained purified crystalline product after work-up. Purity is given in weight percent of the obtained solid.

### Example 1: Sulfinylation of 5-amino-1-[2,6-dichloro-4-(trifluoromethyl)phenyl]-1H-pyrazole-3-carbonitrile with triethylamine hydrochloride, potassium trifluoromethylsulfinate and thionylchloride, in 4.5 molar equivalents of toluene at 35°C

Within a 3-neck, 50 mL round bottom flask equipped with a magnetic stirrer bar and a thermometer were placed vacuum dried potassium trifluoromethylsulfinate (5.16 g, 30 mmol), vacuum dried triethylamine hydrochloride (5.16 g, 37.5 mmol), and 10.4 g anhydrous toluene (4.5 molar equivalents relative to 5-amino-1-[2,6-dichloro-4-(trifluoromethyl)phenyl]-1H-pyrazole-3-carbonitrile) under an argon atmosphere. After cooling to 0°C to 5 °C with external cooling, thionylchloride (3.57 g, 30 mmol) was added within 15 min while keeping the reaction temperature below 5 °C. After stirring for another 30 min, vacuum dried 5-amino-1-(2,6-dichloro-4-trifluoromethyl-phenyl)-1H-pyrazole-3-carbonitrife (8.03 g, 25 mmol, 99 % purity) was added at 5°C, and the reaction mixture was kept at 5 °C for 60 min and then heated to 35 °C within 45 min. The temperature of 35 °C was kept for 3 hours before adding 4.6 g of toluene. After another 7 hours at 35 °C the reaction was quenched with 20 g of sodium hydroxide solution (10 wt.%).

The resulting suspension was diluted with 30 mL of ethylacetate. After phase separation the organic layer was washed once with sodium hydroxide solution (10 wt.%). After phase separation, the organic layer was analyzed by quantitative HPLC (80.4 % yield). The content of compound F was below 4.9 weight percent in the crude mixture (without solvent). The product was crystallized from a mixture of ethylacetate and toluene affording the title compound as a white crystalline powder (7.98 g, 73 % yield, 98 % purity by quantitative HPLC).

### Example 2: Sulfinylation of 5-amino-1-[2,6-dichloro-4-(trifluoromethyl)phenyl]-1H-pyrazole-3-carbonitrile with triethylamine hydrochloride, potassium trifluoromethylsulfinate and thionylchloride, in 6.5 molar equivalents of toluene at 35°C

Within a 750 mL reactor with a mechanical stirrer and a thermometer were placed vacuum dried potassium trifluoromethylsulfinate (50.4 g, 296 mmol), vacuum dried triethylamine hydrochloride (51.1 g, 368 mmol), and 147 g anhydrous toluene (6.5 molar equivalents relative to 5-amino-1-[2,6-dichloro-4-(trifluoromethyl)phenyl]-1H-pyrazole-3-carbonitrile) under an argon atmosphere. After cooling to 0°C to 5 °C with external cooling, thionylchloride (35.7 g, 294 mmol) was added within 15 min while keeping the reaction temperature below 5 °C. After stirring for another 30 min, vacuum dried 5-amino-1-(2,6-dichloro-4-trifluoromethyl-phenyl)-1H-pyrazole-3-carbonitrile (79.5 g, 245 mmol, 99 % purity) was added at 5°C, and the reaction mixture was kept at 5 °C for 60 min and then heated to 35°C within 45 min. The temperature was kept for another 10 hours before quenching the reaction with 200 g of sodium hydroxide solution (10 wt.%).

The resulting suspension was diluted with 176 mL of ethylacetate. After phase separation the organic layer was washed once with sodium hydroxide solution (10 wt.%). After phase separation, the organic layer was analyzed by quantitative HPLC, giving the yield of non-isolated fipronil.

Comparative example C1: Sulfinylation of 5-amino-1-[2.6-dichloro-4-(trifluoromethyl)phenyl]-1H-pyrazole-3-carbonitrile with triethylamine hydrochloride, potassium trifluoromethylsulfinate and thionylchloride, in 6.5 molar equivalents of toluene at 45°C

Comparative example C1 was conducted according to the procedure described in example 2, but the reaction mixture was kept at 5 °C for 60 min and then heated to 45°C instead of 35°C within 45 min.

| Example | Reaction temperature | non-isolated yield Fipronil | compound F w/w % without solvents |
|---|---|---|---|
| 2 | 35 °C | 78.9% | 3.0% |
| C1 | 45 °C | 72.3% | 6.0% |

### Example 3: Sulfinylation of 5-amino-1-12,6-dichloro-4-(trifluoromethyl)phenyl]-1H-pyrazole-3-carbonitrile with thionylchloride, triethylamine hydrochloride and dosage of potassium trifluoromethylsulfinate

Within a 750 mL reactor with a mechanical stirrer and a thermometer were placed vacuum dried triethylamine hydrochloride (51.1 g, 368 mmol), 147 g anhydrous toluene (6.5 molar equivalents relative to 5-amino-1-[2,6-dichloro-4-(trifluoromethyl)phenyl]-1H-pyrazole-3-carbonitrile), and thionylchloride (35.7 g, 294 mmol) under an argon atmosphere. After cooling to 0°C to 5 °C with external cooling, vacuum dried potassium trifluoromethylsulfinate (50.4 g, 296 mmol) was added in three equal portions every 10 min while keeping the reaction temperature below 5 °C. After stirring for another 30 min, vacuum dried 5-amino-1-(2,6-dichloro-4-trifluoromethyl-phenyl)-1H-pyrazole-3-carbonitrile (79.5 g, 245 mmol, 99 % purity) was added at 5°C, and the reaction mixture was kept at 5 °C for 60 min and then heated to 35 °C within 45 min. The temperature of 35 °C was kept for another 10 hours before quenching the reaction with 200 g of sodium hydroxide solution (10 wt.%).

The resulting suspension was diluted with 176 mL of ethylacetate. After phase separation the organic layer was washed once with sodium hydroxide solution (10 wt.%). After phase separation, the organic layer was analyzed by quantitative HPLC (86 % yield). The content of compound F was below 2.5 weight percent in the crude mixture (without solvent). The product was crystallized from a mixture of ethylacetate and toluene affording the title compound as a white crystalline powder (80.3 g, 75 % yield, 98 % purity by quantitative HPLC).

### Example 4: Sulfinylation of 5-amino-1-[2,6-dichloro-4-(trifluoromethyl)phenyl]-1H-pyrazole-3-carbonitrile with thionylchloride, morpholine tosylate and dosage of potassium trifluoromethylsulfinate

The preparation was conducted as described for example 3 above. After phase separation, the organic layer was analyzed by quantitative HPLC (83 % yield). The content of compound F was below 0.5 weight percent in the crude mixture (without solvent).

The product was crystallized from toluene affording the title compound as a white crystalline powder (67 g, 72 % yield, 98 % purity by quantitative HPLC).

Comparative example C2 was conducted according to the procedure described in example 4, but the reaction mixture was kept at 5 °C for 60 min and then heated to 50°C instead of 35°C within 45 min. The product was crystallized from toluene affording the title compound as a white crystalline powder (69 % yield, 98 % purity by quantitative HPLC). The example was repeated 2 times.

| Example | Reaction temperature | non-isolated yield Fipronil | compound F w/w % without solvents |
|---|---|---|---|
| 4 | 35 °C | 83 % | 0.5 % |
| C2 | 50°C | 72-79 % | 0.9-1.4% |

## Claims

1. Process for the sulfinylation of a pyrazole derivative, **characterized in that** 5-amino-1-[2,6-dichloro-4-(trifluoromethyl)phenyl]-1H-pyrazole-3-carbonitrile (II) is reacted with a sulfinylating agent S in the presence of at least one amine acid complex wherein the amine(s) are selected from secondary and/or tertiary amines and the acid(s) are selected from hydrochloric acid, hydrofluoric acid, hydrobromic acid, hydroiodic acid, p-toluenesulfonic acid, benzenesulfonic acid, 4-ethyl benzenesulfonic acid, 4-chlorobenzenesulfonic acid, xylene sulfonic acid, 2,3-dimethylbenzene sulfonic acid, 2,4-dimethylbenzene sulfonic acid, 2,5-dimethylbenzene sulfonic acid, 2,6-dimethylbenzene sulfonic acid, 1-napthalenesulfonic acid, 2-napthalenesulfonic acid, mixtures of two or more of the isomers of dimethylbenzene sulfonic acid, mesitylene sulfonic acid, methanesulfonic acid, camphor sulfonic acid, and trifluoromethylsulfonic acid, and with the addition of a halogenating agent, wherein
S is [CF₃S(O)]₂O; or
CF₃S(O)X wherein
X means fluoro, chloro, bromo, iodo, a hydroxy group, or an alkaline or alkaline earth metal salt of the hydroxy group; or
mixtures thereof,
wherein the temperature of the reaction mixture at no time exceeds 39°C.

2. Process according to claim 1 wherein the halogenating agent is selected from thionylchloride, thionylbromide, phosphoroxychloride, oxalylchloride, phosgen, triphosgen ((CC)₃)₂C(=O)), chloroformiates, phosphorpentachloride, phosphortrichloride, trichloromethylchloromethanoat, and xylenesulfonic acid chloride.

3. Process according to claims 1 or 2 wherein the amine of the amine acid complex is selected from
the tertiary alkyl amines trimethylamine, triethylamine, tripropylamine, triisopropylamine, tributylamine, dimethyl ethyl amine, diethyl methylamine, dimethyl n-propyl amine, diisopropylethylamine, DBU (1,8-diazabicyclo[5.4.0]undec-7-ene), DBN (1,5-diazabicyclo[4.3.0]non-5-ene), methyl morpholine, ethyl morpholine, N,N-dimethylaniline, methyl piperidine, methyl pyrrolidine, and methyldibenzylamine; and
the tertiary aromatic amines pyridine, DMAP (dimethylaminopyridine), collidine, lutidine, pyrimidine, pyrazine, and piperazine; and
the secondary alkyl amines dimethylamine, diethylamine, dipropylamine, diisopropylamine, dibutylamine, ethylmethylamine, isopropylmethylamine, and isopropylethylamine, and
the cyclic secondary amines piperidine, piperidine which is substituted by C₁-C₈-alkyl or C₁-C₈-haloalkyl, such as 2-methylpiperidine or 4-methylpiperidine, and pyrrolidine, imidazolidine, pyrrole, piperazine, and morpholine.

4. Process according to any of claims 1 to 3 wherein the amine of the amine acid complex is selected from trimethylamine, triethylamine, tripropylamine, triisopropylamine, dimethyl ethyl amine, diethyl methylamine, dimethyl n-propyl amine, diisopropylethylamine, DBU (1,8-diazabicyclo[5.4.0]undec-7-ene), DBN (1,5-diazabicyclo[4.3.0]non-5-ene), methyl morpholine, ethyl morpholine, N,N-dimethylaniline, methyl piperidine, methyl pyrrolidine, methyldibenzylamine, pyridine, DMAP (dimethylaminopyridine), collidine, lutidine, pyrimidine, pyrazine, and piperazine.

5. Process according to any of claims 1 to 4 wherein the amine of the amine acid complex is selected from trimethylamine, triethylamine, dimethyl ethyl amine, diethyl methylamine, dimethyl n-propyl amine, methyl morpholine, N,N-dimethylaniline, methyl piperidine, methyl pyrrolidine, methyldibenzylamine, and pyridine.

6. Process according to any of claims 1 to 5 wherein the amine of the amine acid complex is selected from dimethylamine, diethylamine, dipropylamine, diisopropylamine, dibutylamine, ethylmethylamine, isopropylmethylamine, isopropylethylamine, piperidine, piperidine which is substituted by C₁-C₈-alkyl or C₁-C₈-haloalkyl, such as 2-methylpiperidine and 4-methylpiperidine, pyrrolidine, pyrrolidine which is substituted by C₁-C₈-alkyl or C₁-C₈-haloalkyl, such as 2-methylpyrrolidine and 3-methylpyrrolidine, imidazolidine, pyrrole, piperazine, and morpholine.

7. Process according to any of claims 1 to 5 wherein the acid of the amine acid complex is selected from hydrochloric acid, p-toluenesulfonic acid, benzene sulfonic acid and xylene sulfonic acid.

8. Process according to any of claims 1 to 6 wherein the acid of the amine acid complex is selected from p-toluenesulfonic acid, benzenesulfonic acid, 4-ethyl benzenesulfonic acid, 4-chlorobenzenesulfonic acid, xylene sulfonic acid, 2,3-dimethylbenzene sulfonic acid, 2,4-dimethylbenzene sulfonic acid, 2,5-dimethylbenzene sulfonic acid, 2,6-dimethylbenzene sulfonic acid, 1-napthalenesulfonic acid, 2-napthalenesulfonic acid, mixtures of two or more of the isomers of dimethylbenzene sulfonic acid, mesitylene sulfonic acid, methanesulfonic acid, camphor sulfonic acid, and trifluoromethylsulfonic acid, preferably from p-toluenesulfonic acid, benzene sulfonic acid and xylene sulfonic acid.

9. Process according to any of claims 1 to 8 wherein the sulfinylating agent S is selected from CF₃S(O)Cl, CF₃S(O)OH, [CF₃S(O)]₂O, CF₃S(O)ONa, CF₃S(O)OK, and mixtures thereof.

10. Process according to any of claims 1 to 9 wherein the reaction is conducted in an organic solvent selected from toluene, benzene, xylene, trifluoromethylbenzene, monochlorobenzene, ethylbenzene and dichlorobenzene.

11. Process according to any of claims 1 to 10 wherein 1.4 to 2.2 molar equivalents of the amine acid complex relative to 5-amino-1-[2,6-dichloro-4-(trifluoromethyl)phenyl]-1H-pyrazole-3-carbonitrile are used.

12. Process according to any of claims 1 to 11 wherein 1.0 to 1.35 molar equivalents of the sulfinylating agent relative to 5-amino-1-[2,6-dichloro-4-(trifluoromethyl)phenyl]-1H-pyrazole-3-carbonitrile are used.

13. Process according to any of claims 1 to 12 wherein the reaction product 5-amino-1-[2,6-dichloro-4-(trifluoramethyl)phenyl]-4-(trifluoromethylsulfinyl)-pyrazole-3-carbonitrile is crystallized from a solution of monochlorobenzene, dichlorobenzene, ethylbenzene or toluene.

14. Process according to any of claims 1 to 13 wherein in a further step, 5-amino-1-[2,6-dichloro-4-(trifluoromethyl)phenyl]-4-(trifluoromethylsulfinyl)-pyrazole-3-carbonitrile is formulated into a pesticidal composition.

15. Process according to any of claims 1 to 14 wherein in a further step, 5-amino-1-[2,6-dichloro-4-(trifluoromethyl)phenyl]-4-(trifluoromethylsulfinyl)-pyrazole-3-carbonitrile, its veterinarily acceptable enantiomers or salts are converted into a parasiticidal composition.

## Patentansprüche

1. Verfahren zur Sulfinylierung eines Pyrazolderivats, **dadurch gekennzeichnet, dass** man 5-Amino-1-[2,6-dichlor-4-(trifluormethyl)phenyl]-1H-pyrazol-3-carbonitril (II) in Gegenwart mindestens eines Amin-Säure-Komplexes, wobei man das Amin bzw. die Amine aus sekundären und/oder tertiären Aminen auswählt und die Säure bzw. die Säuren aus Chlorwasserstoffsäure, Fluorwasserstoffsäure, Bromwasserstoffsäure, Iodwasserstoffsäure, p-Toluolsulfonsäure, Benzolsulfonsäure, 4-Ethylbenzolsulfonsäure, 4-Chlorbenzolsulfonsäure, Xylolsulfonsäure, 2,3-Dimethylbenzolsulfonsäure, 2,4-Dimethylbenzolsulfonsäure, 2,5-dimethylbenzolsulfonsäure, 2,6-Dimethylbenzolsulfonsäure, 1-Napthalinsulfonsäure, 2-Napthalinsulfonsäure, Gemischen von zwei oder mehr der Isomere von Dimethylbenzolsulfonsäure, Mesitylensulfonsäure, Methansulfonsäure, Camphersulfonsäure und Trifluormethylsulfonsäure auswählt, und unter Zusatz eines Halogenierungsmittels mit einem Sulfinylierungsmittel S umsetzt, wobei S für [CF₃S(O)]₂O oder
CF₃S(O)X, worin
X Fluor, Chlor, Brom, Iod, eine Hydroxygruppe oder ein Alkali- oder Erdalkalimetallsalz der Hydroxygruppe bedeutet;
oder
Mischungen davon steht,
wobei die Temperatur der Reaktionsmischung zu keinem Zeitpunkt 39°C überschreitet.

2. Verfahren nach Anspruch 1, bei dem man das Halogenierungsmittel aus Thionylchlorid, Thionylbromid, Phosphoroxidchlorid, Oxalylchlorid, Phosgen, Triphosgen ((CCl₃)₂C(=O)), Chlorameisensäureestern, Phosphorpentachlorid, Phosphortrichlorid, Chlormethansäuretrichlormethylester und Xylolsulfonsäurechlorid auswählt.

3. Verfahren nach einem der Ansprüche 1 oder 2, bei dem man das Amin des Amin-Säure-Komplexes aus den tertiären Alkylaminen Trimethylamin, Triethylamin, Tripropylamin, Triisopropylamin, Tributylamin, Dimethylethylamin, Diethylmethylamin, Dimethyl-n-propylamin, Diisopropylethylamin, DBU (1,8-Diazabicyclo[5.4.0]undec-7-en), DBN (1,5-Diazabicyclo[4.3-0]non-5-en), Methylmorpholin, Ethylmorpholin, N,N-Dimethylanilin, Methylpiperidin, Methylpyrrolidin und Methyldibenzylamin; und
den tertiären aromatischen Aminen Pyridin, DMAP (Dimethylaminopyridin), Collidin, Lutidin, Pyrimidin, Pyrazin und Piperazin; und
den sekundären Alkylamine Dimethylamin, Dimethylamin, Dipropylamin, Diisopropylamin, Dibutylamin, Ethylmethylamin, Isopropylmethylamin und Isopropylethylamin; und
den cyclischen sekundären Aminen Piperidin, Piperidin, das durch C₁-C₈-Alkyl oder C₁-C₈-Halogenalkyl substituiert ist, wie 2-Methylpiperidin oder 4-Methylpiperidin, und Pyrrolidin, Imidazolidin, Pyrrol, Piperazin und Morpholine
auswählt.

4. Verfahren nach einem der Ansprüche 1 bis 3, bei dem man das Amin des Amin-Säure-Komplexes aus Trimethylamin, Triethylamin, Tripropylamin, Triisopropylamin, Dimethylethylamin, Diethylmethylamin, Dimethyl-n-propylamin, Diisopropylethylamin, DBU (1,8-Diazabicyclo[5.4.0]undec-7-en), DBN (1,5-Diazabicyclo[4.3.0]non-5-en), Methylmorpholin, Ethylmorpholin, N,N-Dimethylanilin, Methylpiperidin, Methylpyrrolidin, Methyldibenzylamin, Pyridin, DMAP (Dimethylaminopyridin), Collidin, Lutidin, Pyrimidin, Pyrazin und Piperazin auswählt.

5. Verfahren nach einem der Ansprüche 1 bis 4, bei dem man das Amin des Amin-Säure-Komplexes aus Trimethylamin, Triethylamin, Dimethylethylamin, Diethylmethylamin, Dimethyl-n-propylamin, Methylmorpholin, N,N-Dimethylanilin, Methylpiperidin, Methylpyrrolidin, Methyldibenzylamin und Pyridin auswählt.

6. Verfahren nach einem der Ansprüche 1 bis 5, bei dem man das Amin des Amin-Säure-Komplexes aus Dimethylamin, Dimethylamin, Dipropylamin, Diisopropylamin, Dibutylamin, Ethylmethylamin, Isopropylmethylamin, Isopropylethylamin, Piperidin, Piperidine das durch C₁-C₈-Alkyl oder C₁-C₈-Halogenalkyl substituiert ist, wie 2-Methylpiperidin und 4-Methylpiperidin, Pyrrolidin, Pyrrolidin, das durch C₁-C₈-Alkyl oder C₁-C₈-Halogenalkyl substituiert ist, wie 2-Methylpyrrolidin und 3-Methylpyrrolidin, Imidazolidin, Pyrrol, Piperazin und Morpholin auswählt.

7. Verfahren nach einem der Ansprüche 1 bis 5, bei dem man die Säure des Amin-Säure-Komplexes aus Chlorwasserstoffsäure p-Toluolsulfonsäure, Benzolsulfonsäure und Xylolsulfonsäure auswählt.

8. Verfahren nach einem der Ansprüche 1 bis 6, bei dem man die Säure des Amin-Säure-Komplexes aus p-Toluolsulfonsäure, Benzolsulfonsäure, 4-Ethylbenzolsulfonsäure, 4-Chlorbenzolsulfonsäure, Xylolsulfonsäure, 2,3-Dimethylbenzolsulfonsäure, 2,4-Dimethylbenzolsulfonsäure, 2,5-Dimethylbenzolsulfonsäure, 2,6-Dimethylbenzolsulfonsäure, 1-Naphthalinsulfonsäure, 2-Naphthalinsulfonsäure, Gemischen von zwei der mehr der Isomere von Dimethylbenzolsulfonsäure, Mesitylensulfonsäure, Methansulfonsäure, Camphersulfonsäure und Trifluormethylsulfonsäure, vorzugsweise aus p-Toluolsulfonsäure, Benzolsulfonsäure und Xylolsulfonsäure, auswählt.

9. Verfahren nach einem der Ansprüche 1 bis 8, bei dem man das Sulfinylierungsmittel S aus CF₃S(O)Cl, CF₃S(O)OH, [CF₃S(O)]₂O, CF₃S(O)ONa, CF₃S(O) OK und Mischungen davon auswählt.

10. Verfahren nach einem der Ansprüche 1 bis 9, bei dem man die Umsetzung in einem aus Toluol, Benzol, xylol, Trifluormethylbenzol, Monochlorbenzol, Ethylbenzol und Dichlorbenzol ausgewählten organischen Lösungsmitten durchführt.

11. Verfahren nach einem der Ansprüche 1 bis 10, bei dem man 1,4 bis 2,2 Moläquivalente des Amin-Säure-Komplexes, bezogen auf 5-Amino-1-[2,6-dichlor-4-(trifluormethyl)phenyl]-1H-pyrazol-3-carbonitril, verwendet.

12. Verfahren nach einem der Ansprüche 1 bis 11, bei dem man 1,0 bis 1,35 Moläquivalente des Sulfinylierungsmittels, bezogen auf 5-Amino-1-[2,6-dichlor-4-(trifluormethyl)phenyl]-1H-pyrazol-3-carbonitril, verwendet.

13. Verfahren nach einem der Ansprüche 1 bis 12, bei dem man das Reaktionsprodukt 5-Amino-1-[2,6-dichlor-4-(trifluormethyl)phenyl]-4-(trifluormethylsulfinyl)pyrazol-3-carbonitril aus einer Lösung von Monochlorbenzol, Dichlorbenzol, Ethylbenzol oder Toluol kristallisiert.

14. Verfahren nach einem der Ansprüche 1 bis 13, bei dem man in einem weiteren Schritt 5-Amino-1-[2,6-dichlor-4-(trifluormethyl)phenyl]-4-(trifluormethylsulfinyl)pyrazol-3-carbonitril zu einem pestiziden Mittel formuliert.

15. Verfahren nach einem der Ansprüche 1 bis 14, bei dem man in einem weiteren Schritt 5-Amino-1-[2,6-dichlor-4-(trifluormethyl)phenyl]-4-(trifluormethylsulfinyl)pyrazol-3-carbonitril oder dessen veterinärmedizinisch unbedenkliche Enantiomere oder Salze in ein parasitizides Mittel umwandelt.

## Revendications

1. Procédé de sulfinylation d'un dérivé de pyrazole, **caractérise en ce que** du 5-amino-1-[2,6-dichloro-4-(trifluorométhyl)phényl-1H-pyrazole-3-carbonitrile (II) est réagi avec agent de sulfinylation S en présence d'au moins un complexe aminé-acide où la ou les amines sont choisies parmi les aminés secondaires et/ou tertiaires, et le ou les acides sont choisis parmi l'acide chlorhydrique, l'acide fluorhydrique, l'acide bromhydrique, l'acide iodhydrique, l'acide p-toluènesulfonique, l'acide benzènesulfonique, l'acide 4-éthylbenzènesulfonique, l'acide 4-chlorobenzènesulfonique, l'acide xylènesulfonique, l'acide 2,3-diméthylbenzènesulfonique, l'acide 2,4-diméthylbenzènesulfonique, l'acide 2,5-diméthylbenzènesulfonique, l'acide 2,6-diméthylbenzènesulfonique, l'acide 1-naphtalènesulfonique, l'acide 2-naphtalènesulfonique, des mélanges de deux, ou plus, des isomères de l'acide diméthylbenzènesulfonique, l'acide mésitylènesulfonique, l'acide méthanesulfonique, l'acide camphresulfonique, et l'acide trifluorométhylsulfonique, et avec l'addition d'un agent d'halogénation, où
S est [CF₃S(O)]₂O ou CF₃S(O)X où X est fluoro, chloro, bromo, iodo, un groupement hydroxy, ou un sel de métal alcalin ou alcalino-terreux du groupement hydroxy ; ou des mélanges de ceux-ci,
la température du mélange réactionnel n'excédant à aucun moment 39°C.

2. Procédé selon la revendication 1, dans lequel l'agent d'halogénation est choisi parmi le chlorure de thionyle, le bromure de thionyle, l'oxychlorure de phosphore, le chlorure d'oxalyle, le phosgène, le triphosgène ((CCl₃)₂C(=O)), les chloroformiates, le pentachlorure de phosphore, le trichlorure de phosphore, le chlorométhanoate de trichlorométhyle, et le chlorure d'acide xylènesulfonique.

3. Procédé selon la revendication 1 ou 2, dans lequel l'aminé du complexe aminé-acide est choisie parmi les alkylamines tertiaires, la triméthylamine, la triéthylamine, la tripropylamine, la triisopropylamine, la tributylamine, la diméthyléthylamine, la diéthylméthylamine, la diméthyl-n-propylamine, la diisopropyléthylamine, le DBU (1,8-diazabicyclo[5,4,0]undéc-7-ène), le DBN (1,5-diazabicyclo[4,3,0]non-5-ène), la méthylmorpholine, l'éthylmorpholine, la N-N-diméthylaniline, la méthylpipéridine, la méthylpyrrolidine, et la méthyldibenzylamine ; et
les amines aromatiques tertiaire, la pyridine, la DMAP (diméthylaminopyridine), la collidine, la lutidine, la pyrimidine, la pyrazine, et la pipérazine ; et
les alkylamines secondaires, la diméthylamine, la diéthylamine, la dipropylamine, la diisopropylamine, la dibutylamine, l'éthylméthylamine, l'isopropylméthylamine, et l'isopropyléthylamine, et les aminés secondaires cycliques, la pipéridine, la pipéridine qui est substituée par C₁-C₈ ou halogénoalkyle en C₁-C₈, telle que la 2-méthylpipéridine ou la 4-méthylpipéridine, et la pyrrolidine, l'imidazolidine, le pyrrole, la pipérazine et la morpholine.

4. Procédé selon l'une quelconque des revendications 1 à 3, dans lequel l'aminé du complexe amine-acide est choisi parmi la triméthylamine, la triéthylamine, la tripropylamine, la triisopropylamine, la diméthyléthylamine, la diéthylméthylamine, la diméthyl-n-propylamine, la diisopropyléthylamine, le DBU (1,8-diazabicyclo[5,4,0]undéc-7-ène), le DBN (1,5-diazabicyclo[4,3,0]non-5-ène), la méthylmorpholine, l'éthylmorpholine, la N,N-diméthylaniline, la méthylpipéridine, la méthylpyrrolidine, la méthyldibenzylamine, la pyridine, la DMAP (diméthylaminopyridine), la collidine, la lutidine, la pyrimidine, la pyrazine, et la pipérazine.

5. Procédé selon l'une quelconque des revendications 1 à 4, dans lequel l'aminé du complexe amine-acide est choisi parmi la triméthylamine, la triéthylamine, la diméthyléthylamine, la diéthylméthylamine, la diméthyl-n-propylamine, la méthylmorpholine, la N,N-diméthylaniline, la méthylpipéridine, la méthylpyrrolidine, la méthyldibenzylamine, et la pyridine.

6. Procédé selon l'une quelconque des revendications 1 à 5, dans lequel l'amine du complexe amine-acide est choisi parmi la diméthylamine, la diéthylamine, la dipropylamine, la diisopropylamine, la dibutylamine, l'éthylméthylamine, l'isopropylméthylamine, l'isopropyléthylamine, la pipéridine, la pipéridine qui est substituée par alkyle en C₁-C₈ ou halogénoalkyle en C₁-C₈, telle que la 2-méthylpipéridine ou la 4-méthylpipéridine, et la pyrrolidine, la pyrolidine qui est substituée par alkyle en C₁-C₈ ou halogénoalkyle en C₁-C₈, telle que la 2-méthylpyrrolidine et la 3-méthylpyrrolidine, l'imidazolidine, le pyrrole, la pipérazine et la morpholine.

7. Procédé selon l'une quelconque des revendications 1 à 5, dans lequel l'acide du complexe amine-acide est choisi parmi l'acide chlorhydrique, l'acide p-toluènesulfonique, l'acide benzènesulfonique, et l'acide xylènesulfonique.

8. Procédé selon l'une quelconque des revendications 1 à 6, dans lequel l'acide du complexe amine-acide est choisi parmi l'acide p-toluènesulfonique, l'acide benzènesulfonique, l'acide 4-éthylbenzènesulfonique, l'acide 4-chlorobenzènesulfonique, l'acide xylènesulfonique, l'acide 2,3-diméthylbenzènesulfonique, l'acide 2,4-diméthylbenzènesulfonique, l'acide 2,5-diméthylbenzènesulfonique, l'acide 2,6-diméthylbenzènesulfonique, l'acide 1-naphtalènesulfonique, l'acide 2-naphtalènesulfonique, des mélanges de deux, ou plus, des isomères de l'acide diméthylbenzènesulfonique, l'acide mésitylènesulfonique, l'acide méthanesulfonique, l'acide camphresulfonique, et l'acide trifluorométhylsulfonique, de préférence parmi l'acide p-toluènesulfonique, l'acide benzènesulfonique et l'acide xylènesulfonique.

9. Procédé selon l'une quelconque des revendications 1 à 9, dans lequel l'agent de sulfinylation S est choisi parmi CF₃S(O)Cl, CF₃S(O)OH, [CF₃S(O)]₂O, CF₃S(O)ONa, CF₃S(O) OK, et des mélanges de ceux-ci.

10. Procédé selon l'une quelconque des revendications 1 à 9, dans lequel la réaction est effectuée dans un solvant organique choisi parmi le toluène, le benzène, le xylène, le trifluorométhylbenzène, le monochlorobenzène, l'éthylbenzène et le dichlorobenzène.

11. Procédé selon l'une quelconque des revendications 1 à 10, dans lequel de 1,4 à 2,2 équivalents molaires du complexe amine-acide par rapport au 5-amino-1-[2,6-dichloro-4-(trifluorométhyl)phényl]-1H-pyrazole-3-carbonitrile sont utilisés.

12. Procédé selon l'une quelconque des revendications 1 à 11, dans lequel de 1,0 à 1,35 équivalent molaire de l'agent de sulfinylation par rapport au 5-amino-1-[2,6-dichloro-4-(trifluorométhyl)phényl]-1H-pyrazole-3-carbonitrile est utilisé.

13. Procédé selon l'une quelconque des revendications 1 à 12, dans lequel le produit de réaction de 5-amino-1-[2,6-dichloro-4-(trifluorométhyl)phényl]-4-(trifluorométhylsulfinyl)pyrazole-3-carbonitrile est cristallisé dans une solution de monochlorobenzène, de dichlorobenzène, d'éthylbenzène ou de toluène.

14. Procédé selon l'une quelconque des revendications 1 à 13, dans lequel, dans une étape supplémentaire, le 5-amino-1-[2,6-dichloro-4-(trifluorométhyl)phenyl]-4-(trifluorométhylsulfinyl)pyrazole-3-carbonitrile est formulé en une composition pesticide.

15. Procédé selon l'une quelconque des revendications 1 à 14, dans lequel, dans une étape supplémentaire, le 5-amino-1-[2,6-dichloro-4-(trifluorométhyl)phényl]-4-(trifluorométhylsulfinyl-pyrazole-3-carbonitrile, ses énantiomères ou sels acceptables sur le plan vétérinaire, sont convertis en une composition parasiticide.
